Europäisches Patentamt

European Patent Office

Office européen des brevets

(11) Veröffentlichungsnummer: **0 369 322**
**A2**

## (12) EUROPÄISCHE PATENTANMELDUNG

(21) Anmeldenummer: **89120767.2**

(22) Anmeldetag: **09.11.89**

(51) Int. Cl.5: **A61M 5/14**

(30) Priorität: **12.11.88 DE 3838465**

(43) Veröffentlichungstag der Anmeldung:
**23.05.90 Patentblatt 90/21**

(84) Benannte Vertragsstaaten:
**DE ES FR GB IT SE**

(71) Anmelder: **Fresenius AG**
**Gluckensteinweg 5**
**D-6380 Bad Homburg v.d.H.(DE)**

(72) Erfinder: **Metzner, Dieter, Dipl.-Ing.**
**Am Geheegberg 23**
**D-8720 Schweinfurt(DE)**

(74) Vertreter: **Görtz, Dr. Fuchs, Dr. Luderschmidt**
**Patentanwälte**
**Abraham-Lincoln-Strasse 7 Postfach 46 60**
**D-6200 Wiesbaden(DE)**

(54) **Spritzenpumpe.**

(57) Um ein Ein- und Ausrasten der Antriebselemente einer Spritzenpumpe zu vermeiden, ist ein Kupplungselement (17) vorgesehen, das permanent mit der Gewindespindel (14) im Eingriff ist. Das Kupplungselement (17), das aus zwei Zahnrädern (18a, 18b) oder aus einer Mutter bestehen kann, wird über Bremselemente (21) blockiert bzw. freigegeben. Die Bremselemente (21) werden über ein Bremsrohr (15) und einen Gelenkhebel (24) von einem Betätigungsknopf (9) betätigt. Durch den permanenten Eingriff des Kupplungselementes (17) mit der Gewindespindel (14) treten zu Beginn des Pumpvorganges keine mechanischen Spiele auf.

FIG. 3

## Spritzenpumpe

Die Erfindung betrifft eine Spritzenpumpe zur dosierenden Förderung einer Flüssigkeit aus einer Injektionsspritze mit einem Gehäuse, das eine Halterung für die Spritze aufweist, mit einem motorischen Antrieb, mit einem Schraubenspindelgetriebe zur Übertragung der motorischen Antriebskraft vom Antrieb auf den Spritzenkolben, mit einem Antriebselement, das an einem an der Druckplatte des Spritzenkolbens angreifenden Schieber befestigt ist, und mit einem an dem Antriebselement angeordneten und an der Schraubenspindel angreifenden Kupplungselement, das drehbar am Antriebselement angeordnet und zum permanenten Eingriff in das Gewinde der Spindel ausgebildet ist, wobei ein zum Blockieren und Freigeben des Kupplungselementes bewegliches Bremselement vorgesehen ist, das über ein federbelastetes Gestänge mit einem Betätigungselement verbunden ist, wobei sich ein Ende der Feder des Gestänges am Schieber abstützt.

Spritzenpumpen werden im klinischen Bereich und in der medizinischen Forschung verwendet, um dem Patienten kleinere Flüssigkeitsmengen über einen längeren Zeitraum zu applizieren.

Eine derartige Spritzenpumpe ist beispielsweise aus der US-A 4,191,187 bekannt. Bei dieser Spritzenpumpe weist das Schraubenspindelgetriebe eine axial gehäusefest angeordnete Gewindespindel auf, auf der ein Lagerblock angeordnet ist, in dem ein Kupplungsbolzen angeordnet ist. Der Kupplungsbolzen weist eine Ausnehmung auf, die innen mit einem Gewindesegment versehen ist, welches mit der Gewindespindel in Eingriff gebracht werden kann. Parallel zur Gewindespindel ist eine Führungsfläche am Gehäuse vorgesehen, an der der Lagerblock verschiebbar geführt ist.

Eine weitere, in der DE-A 34 28 655 beschriebene Spritzenpumpe weist ein Gehäuse auf, das mit einer Halterung für die Spritze versehen ist. Ferner ist ein motorischer Antrieb vorgesehen sowie ein Getriebe zur Übertragung der motorischen Antriebskraft vom Antrieb auf die Spritze, wobei das Getriebe ein Antriebselement aufweist, das mit dem beweglichen Spritzenteil in Eingriff bringbar ist, um dieses translatorisch zu bewegen. Um die Spritze nach Gebrauch auf einfache Weise wieder gebrauchsfertig zu machen, ist eine Kupplung vorgesehen, die ein Ausrückglied aufweist, um das Getriebe vom Antrieb zu trennen. Um eine verkippfreie Führung zu schaffen, ist vorgesehen, daß ein Lagerbock ein längliches Führungsstück trägt, das im Gleitsitz auf der Gewindespindel angeordnet ist und, daß das Ausrückglied der Kupplung am Führungsstück bzw. am Lagerbock verschiebbar gelagert ist.

Ein Ein- bzw. Auskuppeln des Ausrückgliedes wird dabei manuell über ein außerhalb des Gehäuses liegendes Anschlußstück und über das daran befestigte, in das Gehäuse hineinragende und an das Ausrückglied angelenkte Antriebselement vorgenommen.

Diese Kupplungen haben den Nachteil, daß sie beim Einrasten nicht immer genau das gegenüberliegende Gewinde finden und somit Rastkontrolle benötigen.

Beim Zustandekommen des eingerasteten Zustandes kann eine beachtliche Zeit vergehen, bis die weiteren durch die Mechanik bedingten Spiele aufgehoben sind und eine tatsächliche translatorische Bewegung des Lagerbocks beginnt.

Aus der DE-A 34 39 322 ist eine Infusionspumpe bekannt, die einen Freilauf in einer Richtung zulassendes Klinkenrad aufweist, das mit einer Klinke zusammenwirkt. Die Klinke kann nicht unmittelbar betätigt werden, sondern nur indirekt durch Ziehen an eine Handhabe, wodurch eine gewisse Bewegung der Klinke durch das Klinkenrad hervorgerufen wird. Die Anordnung erlaubt jedoch nur eine Bewegung der mit dem Spritzenkolben verbundenen Stange entgegen der Förderrichtung, da in Förderrichtung die Stange durch die besagte Klinke gesperrt wird und diese auch nicht betätigt werden kann, um bei einer Bewegung in Förderrichtung den Bremseingriff zu lösen. Dies soll jedoch bei der aus der DE-A 34 39 322 bekannten Spritzenpumpe auch gar nicht bezweckt werden, da die Klinke dazu vorgesehen ist, ein ungewolltes Eindrücken des Spritzenkolbens allein aufgrund der Federkraft zu verhindern.

Aus der aufgrund dieser Konstruktion notwendigen Wirkungsweise der Klinke ergibt sich jedoch der Nachteil, daß ein bewußtes Einstellen des Kolbens in Förderrichtung, beispielsweise wenn die Spritze nicht vollständig gefüllt ist und der Kolben vor dem eigentlichen Infusionsvorgang an die Flüssigkeitssäule herangeführt werden soll, nicht möglich ist.

Es ist daher Aufgabe der vorliegenden Erfindung, eine Spritzenpumpe der eingangs angegebenen Art zu schaffen, deren Bremselement unmittelbar betätigbar ist, und bei der eine Einstellung des Spritzenkolbens sowohl in als auch entgegen der gewünschten Förderrichtung möglich ist.

Die Lösung dieser Aufgabe erfolgt dadurch, daß das Gestänge ein im Antriebselement geführtes Bremsrohr aufweist, in dem die Feder angeordnet ist, deren anderes Ende mit dem Bremsrohr verbunden ist, und daß das Betätigungselement am Schieber angeordnet ist.

Dadurch, daß bei der erfindungsgemäßen Spritzenpumpe das Bremselement mit Hilfe des Betätigungselementes unmittelbar und unabhängig von einer Bewegung des Antriebselementes gelöst werden kann, ist es möglich, den Spritzenkolben sowohl entgegen als auch in der Förderrichtung der Spritze vor dem eigentlichen Infusionsvorgang zu verstellen.

Vorzugsweise besteht das Antriebselement aus einem die Spindel umgreifenden Zugrohr.

Gemäß einer besonderen Ausführungsform besteht das Kupplungselement aus einem die Spindel kämmenden Zahnrad.

Eine weitere Ausführungsform sieht als Kupplungselement mindestens eine Mutter vor, die am Zugrohr über Kugellager drehbar befestigt ist.

Die erfindungsgemäße Spritzenpumpe hat den Vorteil, daß beim manuellen Verschieben des Antriebselementes längs der Gewindespindel keine Trennung von Gewindespindel und Antriebselement vorgenommen werden muß. Das Kupplungselement, d.h. das Zahnrad oder die Mutter, bleibt ständig im Eingriff mit dem Gewinde der Gewindespindel, so daß nach dem Einsetzen der Injektionsspritze und Fixieren des Schiebers an der Druckplatte der Injektionsspritze keine mechanischen Spiele auftreten, die die tatsächliche translatorische Bewegung des Antriebselementes und des Schiebers verzögern.

Zum manuellen Verschieben des Antriebselementes und des Schiebers wird lediglich das Bremselementgelöst, so daß das Zahnrad bzw. die Mutter freigegeben wird. Das Kupplungselement ist dann in der Lage, während des manuellen Verschiebens auf der Gewindespindel abzurollen oder sich abzuwälzen, wobei der Eingriff mit der Gewindespindel erhalten bleibt. Nach dem Fixieren von Spritze und Druckplatte am Schieber wird das Bremselement betätigt, so daß das Kupplungselement in seiner Lage fixiert wird. Auf diese Weise wird das Antriebselement in seiner Lage bezüglich der Gewindespindel festgelegt, so daß bei der nachfolgenden Drehbewegung der Gewindespindel das Antriebselement in der vorgesehenen Weise bewegt wird. Ein mechanisches Spiel ist zu Beginn der Drehbewegung der Gewindespindel nicht vorhanden, so daß die Bewegung der Gewindespindel unmittelbar auf das Antriebselement und somit auf den Spritzenkolben übertragen wird. Dies führt zu einem Zeitgewinn und einer sicheren Handhabung der Spritzenpumpe.

Damit die Mutter nach dem Lösen des Bremselementes frei um die Gewindespindel rotieren kann, wird die Steigung des Gewindes von Spindel und Mutter so groß gewählt, daß eine Selbsthemmung nicht auftreten kann.

Das Bremselement kann an der Zahnseite des Zahnrades oder aber, wenn eine größere Bremsfläche gewünscht wird, auch an der Stirnseite des Zahnrades angreifen. Wird als Kupplungselement eine Mutter verwendet, greift das Bremselement vorzugsweise an der Stirnseite der Mutter an. Um eine optimale Bremswirkung zu erzielen, ist das Bremselement an seiner Kontaktfläche mit einem Bremsbelag versehen.

Zur Betätigung des Bremselementes ist dieses über ein federbelastetes Gestänge mit einem am Schieber angeordneten Betätigungselement verbunden. Dieses Gestänge kann gemäß einer besonderen Ausführungsform ein im Zugrohr geführtes Bremsrohr aufweisen, in dem eine Feder angeordnet ist, die mit ihrem einen Ende am Bremsrohr und mit ihrem anderen Ende am Schieber befestigt ist. An diesem Bremsrohr ist ein Hebel angelenkt, der mit dem Betätigungsknopf drehbar verbunden ist. Wird der Betätigungsknopf gedrückt, wird das Bremsrohr gegen die Wirkung der Feder bewegt und das Bremselement vom Kupplungselement gelöst. Durch Loslassen des Betätigungsknopfes drückt die Feder das Bremsrohr in die Ausgangsstellung, so daß das Bremselement unter Druck am Kupplungselement anliegt und dieses blockiert.

Zur Aufnahme der Kupplungselemente ist das Antriebselement vorzugsweise mit einem Käfig versehen, der am dem Schieber angewandten Ende des Antriebselementes angeordnet ist.

Beispielhafte Ausführungsformen der Erfindung werden nachfolgenden anhand der Zeichnung näher erläutert. Es zeigen:

Figur 1 eine Draufsicht auf eine Spritzenpumpe,

Figur 2 eine Ansicht der Spritzenpumpe gemäß Figur 1 von unten,

Figur 3 einen Detailschnitt der Kupplung gemäß einer ersten Ausführungsform und

Figur 4 einen Detailschnitt der Kupplung gemäß einer weiteren Ausführungsform.

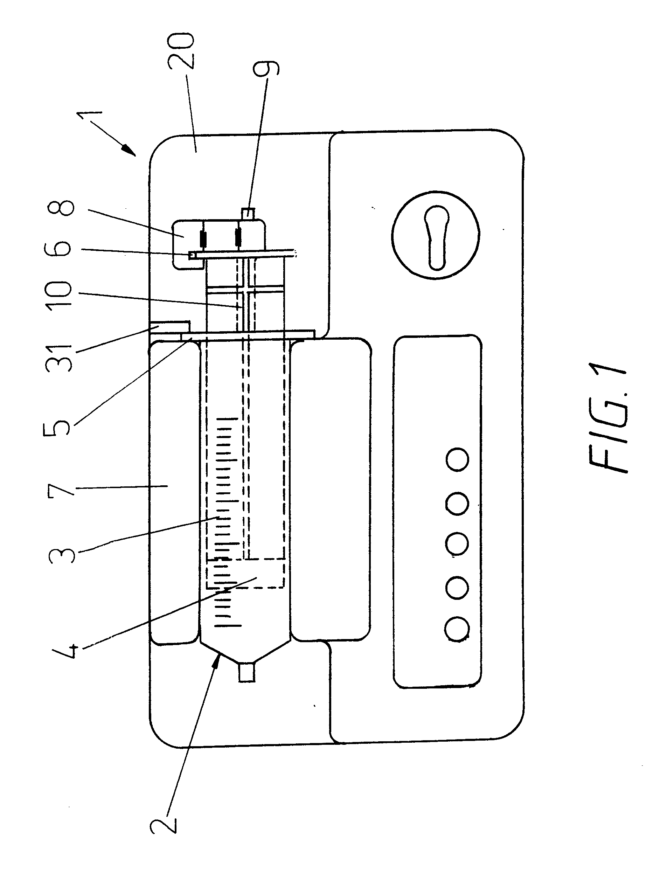

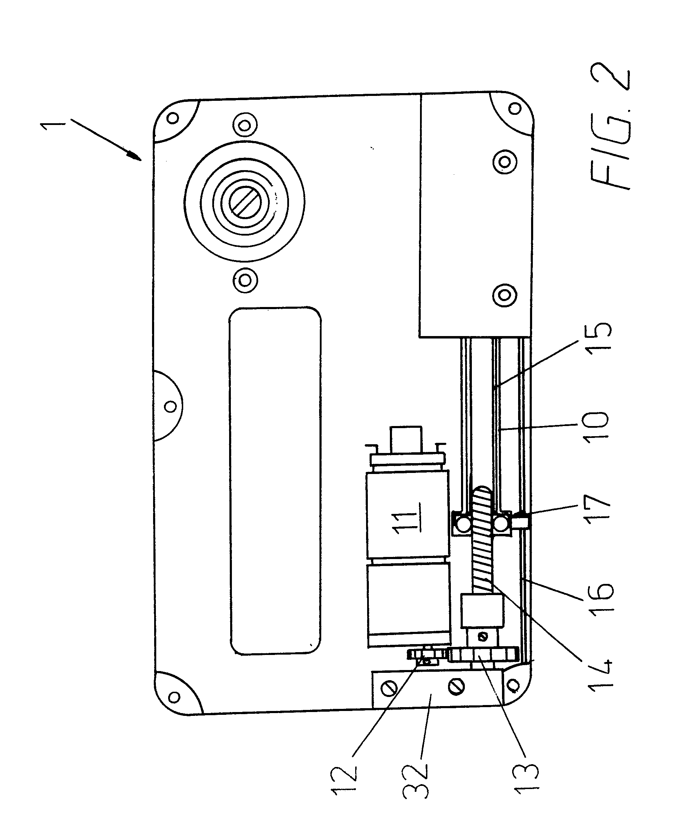

Die in den Figuren 1 und 2 dargestellte Spritzenpumpe 1 weist ein Gehäuse 20 auf, an dessen Oberseite eine den Spritzenkörper 3 am Umfang teilweise umfassende Halterung 7 vorgesehen ist. Mittels einer zwischen einem Halteteil 31 und der Halterung 7 angeordneten Spritzenschulter 5 ist die Spritze 2 in Längsrichtung in der Halterung 7 festgelegt. Der Spritzenkolben 4 ist über die Druckplatte 6 mit dem Schieber 8 verbunden. Dieser Schieber 8 ist wiederum an dem Antriebselement 10 befestigt. Wie in Figur 1 ebenfalls zu sehen ist ist am Schieber 8 ein Knopf 9 zum Betätigen der noch zu beschreibenden Bremse angebracht.

In der Figur 2 ist die Spritzenpumpe 1 von unten dargestellt. Ein motorischer Antrieb 11 ist über die beiden Zahnräder 12 und 13 mit der Gewindespindel 14 verbunden. Als Antrieb 11 kann ein Elektromotor vorgesehen sein, der am Gehäuse 20 befestigt ist.

Die Gewindespindel 14 ist ein Teil des Getriebes, das außer den Zahnrädern 12 und 13 auch noch das Kupplungselement 17 umfaßt. Die Gewindespindel 14 ist parallel zur Motorwelle ausgerichtet, wobei sie mit einem Ende im Kupplungselement 17 drehbar gelagert ist, während das andere Ende von einem an der gegenüberliegenden Gehäusewand befestigten Lagerbock 32 drehbar und in axialer Richtung unverschiebbar gehalten ist.

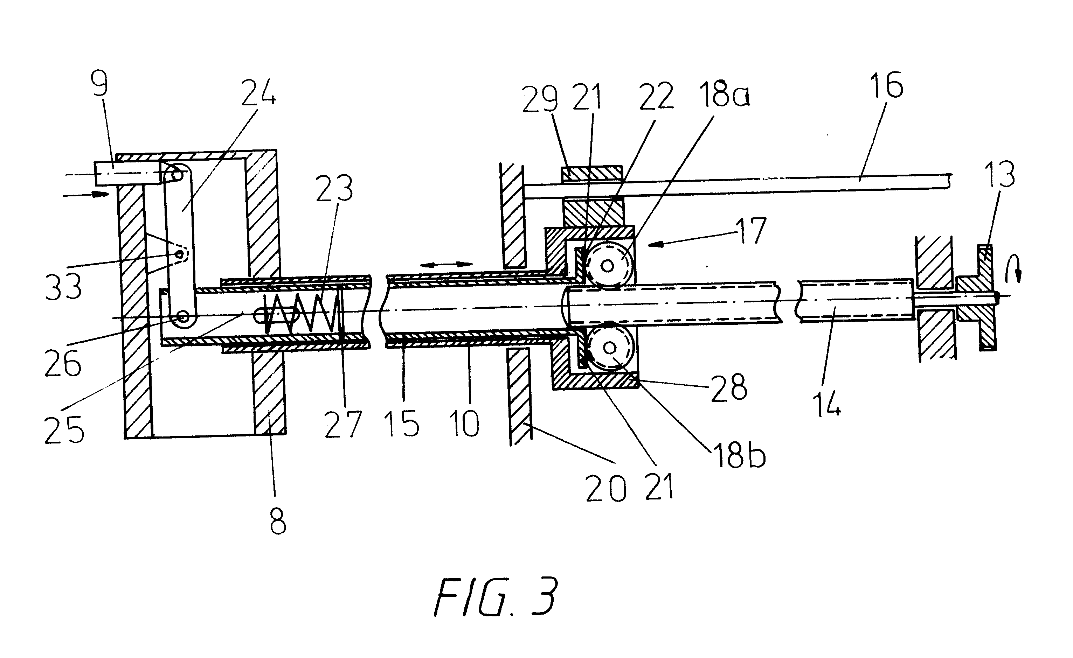

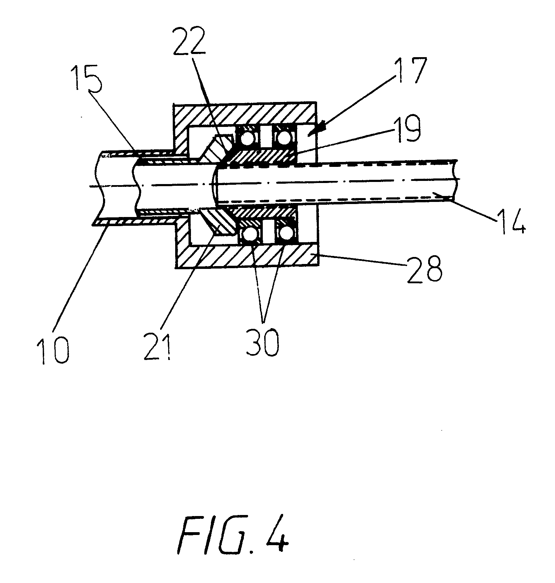

Das Kupplungselement 17, das nachfolgend im Zusammenhang mit den Figuren 3 und 4 noch eingehender beschrieben wird, ist am Antriebselement 10, das als Zugrohr ausgebildet ist, befestigt. Wie in der Figur 3 zu sehen ist, ist das Kupplungselement in einem Käfig 28 untergebracht. Um ein Verdrehen des Zugrohrs 10 während der Rotation der Gewindespindel 14 zu verhindern, ist das Zugrohr 10 bzw. der Käfig 28 über ein Gleitführungselement 29 mit einer Verdrehsicherungsstange 16 verbunden, die parallel zur Gewindespindel am Gehäuse 20 befestigt ist.

In der Figur 3 ist eine der möglichen Ausführungsformen der Kupplung dargestellt. Das Kupplungselement 17 besteht in diesem Beispiel aus den beiden Zahnrädern 18a und 18b, die drehbar im Käfig 28 gelagert sind. Diese Zahnräder 18a, 18b sind mit dem Gewinde der Gewindespindel 14 im permanenten Eingriff. Zum Blockieren und Freigeben der Zahnräder sind zwei Bremselemente 21 vorgesehen, die die Gestalt eines Rohrflansches aufweisen. Die Bremselemente 21 sind einstückig am Bremsrohr 15 angeformt und weisen an ihrer Kontaktfläche einen Bremsbelag 22 auf. Das Bremsrohr ist innerhalb des Zugrohres 10 angeordnet, das am Schieber 8 befestigt ist. Das Bremsrohr 15 reicht ebenfalls bis in das Innere des Schiebers 18 und weist an seinem Ende einen Gelenkhebel 24 auf, der am Bremsrohr 15 im Punkt 26 und am Schieber 8 im Punkt 33 schwenkbar gelagert ist. Am oberen Ende des Gelenkhebels 24 ist der Betätigungsknopf 9 angelenkt. Im Bremsrohr 15 ist weiterhin eine Feder 23 vorgesehen, die mit ihrem einen Ende über die Platte 27 am Bremsrohr befestigt ist. Das andere Ende ist über einen Draht 25 am Schieber 8 befestigt.

Wird der Betätigungsknopf 9 gedrückt, wird das Bremsrohr 15 gegen die Wirkung der Feder 23 nach links verschoben, so daß die Bremselemente 21 die Zahnräder 18a und 18b freigeben. Der Betätigungsknopf 9 wird solange betätigt, bis der Schieber 8 in der gewünschten Position angelangt ist. Hierbei rollen die Zahnräder 18a und 18b über das Gewinde der Spindel 14. Wenn die gewünschte Position erreicht ist, wird der Betätigungsknopf losgelassen, und die Feder 23 drückt das Bremsrohr 15 wieder in die Ausgangsstellung, in der die Bremselemente 21 die Zahnräder 18a und 18b blockieren.

In der Figur 4 ist eine weitere Ausführungsform dargestellt. Das Kupplungselement 17 besteht in diesem Beispiel aus einer Mutter 19, die über die beiden Kugellager 30 im Käfig 28 drehbar gelagert ist. Die Stirnfläche der Mutter 19 ist vorzugsweise schräg ausgebildet, so daß eine größere Kontaktfläche mit dem Bremselement 21 erzielt wird. Dieses Bremselement 21 ist ebenfalls in Gestalt eines Rohrflansches ausgebildet und mit einem Bremsbelag 22 versehen. Die Bremselemente 21 sind am Bremsrohr 15 angeformt, das in der gleichen Weise wie in der Figur 3 beschrieben ist mit dem Betätigungsknopf 9 verbunden ist. Die Wirkungsweise dieses Bremselementes sowie die Betätigung des Bremselementes 21 entspricht der in der Figur 3 dargestellten Weise.

## Ansprüche

1. Spritzenpumpe zur dosierenden Förderung einer Flüssigkeit aus einer Injektionsspritze mit einem Gehäuse, das eine Halterung für die Spritze aufweist, mit einem motorischen Antrieb, mit einem Schraubenspindelgetriebe zur Übertragung der motorischen Antriebskraft vom Antrieb auf den Spritzenkolben, mit einem Antriebselement, das an einem an der Druckplatte des Spritzenkolbens angreifenden Schieber befestigt ist, und mit einem an dem Antriebselement angeordneten und an der Schraubenspindel angreifenden Kupplungselement, das drehbar am Antriebselement angeordnet und zum permanenten Eingriff in das Gewinde der Spindel ausgebildet ist, wobei ein zum Blockieren und Freigeben des Kupplungselementes bewegliches Bremselement vorgesehen ist, das über ein federbelastetes Gestänge mit einem Betätigungselement verbunden ist, wobei sich ein Ende der Feder des Gestänges am Schieber abstützt, dadurch gekennzeichnet, daß das Gestänge ein im Antriebselement (10) geführtes Bremsrohr (15) aufweist, in dem die Feder (23) angeordnet ist, deren anderes Ende mit dem Bremsrohr (15) verbunden ist, und daß das Betätigungselement (9) am Schieber (8) angeordnet ist.

2. Spritzenpumpe nach Anspruch 1, dadurch gekennzeichnet, daß das Antriebselement aus einem die Spindel (14) umgreifenden Zugrohr (10) besteht.

3. Spritzenpumpe nach Anspruch 1 oder 2, dadurch gekennzeichnet, daß das Kupplungselement (17) aus mindestens einem die Spindel (14) kämmenden Zahnrad (18 a, b) besteht.

4. Spritzenpumpe nach Anspruch 1 oder 2, dadurch gekennzeichnet, daß das Kupplungselement (17) aus mindestens einer Mutter (19) besteht, die am Antriebselement über Kugellager (30) drehbar befestigt ist.

5. Spritzenpumpe nach Anspruch 4, dadurch gekennzeichnet, daß die Spindel (14) und die Mutter (19) ein nicht selbsthemmendes Gewinde aufweisen.

6. Spritzenpumpe nach Anspruch 1, dadurch gekennzeichnet, daß das Bremselement (21) an der Stirnseite oder der Zahnseite des Zahnrades (18a, b) angreift.

7. Spritzenpumpe nach einem der Ansprüche 4 oder 5, dadurch gekennzeichnet, daß das Bremselement (21) an der Stirnseite der Mutter (19) angreift.

8. Spritzenpumpe nach einem der Ansprüche 1 bis 7, dadurch gekennzeichnet, daß das Bremselement (21) an seiner Kontaktfläche mit einem Bremsbelag (22) versehen ist.

9. Spritzenpumpe nach einem der Ansprüche 2 bis 8, dadurch gekennzeichnet, daß das Zugrohr (10) an seinem dem Schieber (8) abgewandten Ende einen Käfig (28) zur Aufnahme des Kupplungselements (17) aufweist.

FIG.1

EP 0 369 322 A2

FIG. 2

EP 0 369 322 A2

FIG. 3

FIG. 4